# EUROPEAN PATENT APPLICATION

(11) **EP 1 214 893 A1**
(43) Date of publication of application: **19.06.2002**
(21) Application number: 00127644.3
(22) Date of filing: 16.12.2000
(51) Int. Cl.: A23L 1/30, A61K 35/78

(54) **Health promoting compositions**

(71) Applicant: Aventis Pharma Deutschland GmbH, 65929 Frankfurt am Main (DE)
(72) Inventor: Clayton, Paul, London 2E27 9DZ (GB)

(57) **Abstract**

The invention refers to several compositions promoting human health comprising one or several but not all of the following compounds
a) 800 mcg (2664lU) of Vitamin A, 500 mg of Vitamin C, 15 mcg of Vitamin D, 265 mg (400lU) of Vitamin E, 50 mcg of Vitamin K,
b) 10 mg of Beta carotene, 6 mg of Lutein, 5 mg of Lycopene, 100 mcg of Zeaxanthin,
c) 7.5 mg of Vitamin B1, 7.5 mg of Vitamin B2, 15 mg of Niacin, 15 mg of Pantothenic acid, 7.5 mg of Vitamin B6, 200 mcg of Folic acid, 6.75 mcg of Vitamin B12,
d) 150 mcg of Selenium, 10 mg of Zinc, 100 mg of Calcium, 50 mg of Magnesium,120 mcg of Chromium, 2 mg of Copper, 4 mg of Manganese, 100 mcg of Iodine, 100 mcg of Molybdenum,
e) 200 mcg of Biotin, 450 mg of Betaine, 100 mg of Oligoproanthocyanidins (OPC), 150 mg of Polyphenol complex, 40 mg of Isofloavones in particular genistein and/or daidzein , 600 mg of Omega 3, 4 g of Oligosaccharides (FOS) in particular inulin, and/or oligo-fructose and/or beta glucan, 30-60 mg of Co-Q10,
f) 500 mg of Glucosamine
and
possibly additional substances for the purpose of stabilisation and formulation.

## Description

The invention refers to several compositions of compounds which are useful as food additives or part of pharmaceutical mixtures for improving health problems caused by several diseases.

If free radicals cause illness and premature death, boosting anti-radical defences should improve health and life span.

In animal studies, high doses of anti-oxidants have prolonged life significantly.

An explosion is an extremely fast fire - but humans are an fire too, albeit in a very slow and controlled manner, consuming oxygen and carbon compounds, and burning them to produce water, carbon dioxide, ATP (the energy molecule),and free radicals. The low heat produced in this slow fire keeps us warm and alive.

To stop it from burning out of control and damaging us, we tend the fire very carefully:
we parcel it up into tiny, enzyme-regulated steps, surround it with firebreaks, and place fire extinguishers in every corner to douse the excess free radicals. The 'fire extinguishers' are anti-oxidants.

There are basically three lines of anti-oxidant defences:
1st line Anti-oxidant enzymes are made in the body and contain an atom of selenium, zinc, manganese, copper or iron. Small amounts of these metals are vital to our health.
2nd line Anti-oxidant micro-nutrients are obtained from our diet. These include Vitamins A, C and E, and the S vitamins. Co-enzyme 010, flavancids and carotenaids are important vitamin-5ke compounds which have anti-oxidant properties.
3rd line Anti-oxidant compounds are farmed in the body, and are made up from elements in the diet. These include meiatonin, glutathione, oestrogen, lipoic acid, Q10 and others.

Hardly anyone eats a diet containing enough of ail the anti-oxidant minerals, vitamins and other anti-oxidant compounds. It's important to get enough of all of them, as they work best together in a kind of defence shield. The fact that we don't get enough of them is the main reason why most of us die of free radical-related disease. There are anti-oxidants in every living tissue, in every organ and cell of our body, and in almost every food that we eat. Meat, fish, poultry, milk, egg, vegetables and fruit, nuts, grains and pulses all contain anti-oxidants: if they didn't they would rapidly oxidise and turn rancid on the hoof, or on the vine.

Unfortunately, the major anti-oxidants in meat, milk and eggs are destroyed by cooking. Nor is there much point in ordering raw eggs and steak tartare, because even if the anti-oxidants weren't destroyed by cooking, they would be broken down in the digestive tract.

The anti-oxidants in fruits and vegetables are more likely to survive the cooking process (although you will destroy them eventually if you cook your fruit and vegetables to a pulp, so lightly cooked is best). Anti-oxidants in fruits and vegetables are generally well absorbed - though some people absorb them better than others.

Dietary factors exert an enormous influence on the levels of free radicals in our bodies. But there are many non-dietary lifestyle factors, such as smoking, sunbathing and aerobic exercise, which increase the amount of free radicals in our bodies.

Chronic infection, whether bacterial, viral or fungal, is another cause of increased free radical formation. This too is linked to increased DNA damage and an increased risk of cancer. Some bacteria appear to be more dangerous than others in this respect: helicobacter pylori as a cause of stomach cancer and papilloma virus as a cause of cervical cancer are among the best documented.

The body's first response to increased amounts of free radicals, whatever their origin, is to defend itself by increasing the levels of its anti-oxidant enzymes. This same defence response is found throughout the animal kingdom, and in plants also.

Most dietary advice concentrates on the health benefits of an increased intake of the anti-oxidant vitamins - but the major antioxidant enzymes are diet-dependent too. Each enzyme requires an atom of either zinc, iron, selenium, copper or manganese; and all of these must be obtained from our food. This can be a real problem, because there is good evidence of widespread depletion in one or more of these vital trace elements.

Because micro-nutrient depletion is so prevalent, many of us have sub-optimal anti-radical defences.
There's good evidence that our impaired anti-oxidant defences are a significant cause of ill health - and a good case for saying that our governments should spend less money on treating illness and more on preventing it with better nutritional education. Together with improved food labelling, properly thought-out food fortification programmes and a health-orientated agricultural policy which would shift subsidies from meat and dairy production to fruit and vegetable growers, this would improve the health of the entire nation.

But don't hold your breath. Waiting for governments to make sensible decisions can take a long time, and in this area of policymaking there is strong opposition to change from groups with a vested interest in the status quo.

In the meantime, there's one simple nutritional step we can all take to improve our anti-oxidant defences, which would greatly increase our chances of living a long and healthy life: eat more fruit and vegetables. But even this is no guarantee of achieving optimal nutrition.

With the exception of Co-enzyme Q10, most micro-nutrients are predominantly derived from plant foods, which is why it's good idea to eat more fruit, vegetables, nuts, grains and pulses.
This is not to say that meat is intrinsically unhealthy. The trace anti-oxidant metals, and some anti-oxidant compounds, including the carotenoids, are not exclusive to plants, and can be found in meat too. Unfortunately, they tend to concentrate in parts of the animal that we do not often eat.
Liver and kidney (and testes and brain) are among the best sources of trace metals and of some vitamins like A, K, E and Co-enzyme Q10.
However, offal meats are becoming more unpopular for human consumption, and in many affluent cultures are predominantly used for pet food. The meat we eat is mostly skeletal muscle, which is not as good a source of many micro-nutrients.

Depleted soils produce crops low in specific minerals. This is particularly important in the case of selenium, and is the main cause of the selenium depletion which occurs throughout much of Northern Europe, mainland China, parts of Africa and elsewhere.
If you live in an area where the soil is depleted, or you eat foods imported from regions with depleted soils, or you eat a lot of processed foods, your anti-oxidant enzyme defences are probably sub-optimal. The combination of impaired anti-oxidant defences and increased exposure to free radicals is a recipe for premature aging and illness.

Anti-oxidants work best to prevent disease when given together, rather than as monotherapies. Our foods contain complex mixtures of anti-oxidants, and, before the arrival of the supplement industry, we obtained all our anti-oxidants from food.
For example, many supplements contain beta carotene, but there are over 600 carotenoids in fruits and vegetables, so why supplement with just one?
Although beta carotene is the main carotenoid in most diets, our bodies also contain, and probably need, alpha carotene (from carrots or pumpkins), lutein (green leaf vegetables, especially kale and broccoli), lycopene (tomatoes), cryptoxanthin (oranges), zeaxanthin (red pepper and spinach) and others.
The many studies which have looked at the effects of single anti-oxidants are, in part, an unfortunate hangover from the pharmaceutical mind-set. Despite the headlines, it is not very helpful to single out any one micro-nutrient (such as lycopene or selenium) as being 'anti-cancer': it is the general nutritional status that is important. For example, low selenium has been linked to arthritis and myocardial disease in China, coronary artery disease in Finland, breast cancer in New Zealand, and goitre in Zaire.

Each country has a typical nutritional profile through which the low selenium filters, causing a characteristic pattern of illness in each different territory.
The section at the end of this chapter is an attempt to design anti-oxidant and nutritional programs for specific illnesses; but what about the healthy person who just wants to stay healthy ? Which anti-oxidants should they take ?
Scientists use a number of different tests to measure the anti-oxidant effectiveness of different compounds, but have not yet reached any general agreement as to which test is the most meaningful. This is not an ideal state of affairs, but nevertheless a few general patterns have emerged.
Many tests show that Vitamin C tends to be used up first. When the C is gone, the carotenoids go next, then Vitamin E; and when the E has all been used up, lipid oxidation begins. Adding either C or E will prevent the lipid oxidation but does not protect proteins from oxidative damage. To protect proteins you must add other anti-oxidants such as flavonoids or glutathione (of which more later). So when it comes to anti-oxidants, monotherapy (ie: attempting to prevent free radical damage with a single anti-oxidant) is both theoretically and demonstrably wrong.
Beta carotene - 10-15 mg/day - mixed, natural source carotenoids are best - always combine with Vitamin C.
This dose puts you in the top few per cent of the population, a group which appears to be at reduced risk of oral and colon cancers, coronary artery disease and cataracts. Larger doses are generally safe: 500 patients with skin conditions took 180-300 mg beta carotene/day for 10 years, without adverse effects⁽⁶³⁾. However, smokers and others at risk of lung cancer must be cautious with beta carotene.

It is best to combine beta carotene with other carotenoids: 6 mg lycopene and 6 mg lutein per day is a reasonable level, and beta carotene should always be taken with Vitamin C.

Vitamin E - 40OW (265 mg)/day - natural source is best. The American Physicians Trial and other data suggest this is an optimal dose for coronary disease. It should be natural E: the natural form contains only one isomer, D-alpha, whereas synthetic E contains eight.
Why natural Vitamin E ? Because whereas D-alpha inhibits the proliferation of smooth muscle cells in blood vessel walls (a highly desirable and-coronary effect), some of the other isomers in synthetic E block this property, and could therefore be cardiotoxic. Very high doses of E are probably not generally useful and may be potentially harmful if used without other anti-oxidants. They may also inhibit the absorption of beta carotene and Vitamin K.

The current theory of coronary artery disease centres an the oxidization of LDL (low-density lipoprotein) cholesterol particles in the blood. The resulting lipid and cholesterol oxidation products attack the artery walls, increase the risk of clotting and raise blood pressure - all of which increases the risk of heart attacks.
Many anti-oxidants help protect LDL cholesterol from oxidation. When supplements of selenium and the Vitamins C and E are given to human volunteers, their cholesterol becomes more resistant to oxidation. Vitamin E has been shown to reduce the risk of heart attacks by half or more in three recent trials, and to reduce the progression of atheroma (furring of the arteries) in ways which are now well understood. So should we all be taking Vitamin E ?
Yes, but not on its own. Under certain conditions, Vitamin E can accelerate the oxidation of LDL cholesterol unless there is enough Vitamin C (or flavonoids) around to protect the Vitamin E itself from being oxidized.

This is probably one of the main cardioprotective roles of Vitamin C. However, Vitamin C also helps to maintain the lining of the arteries and reduces levels of clotting factors in the blood. It used to be thought that Vitamin C could increase levels of the 'good' HIDL (high-density lipoprotein) cholesterol, but this now looks unlikely.
All this means that Vitamin E should really be taken with Vitamin C, Co-enzyme G10 (which appears to be a critical antioxidant in LIDL), and the metal trace elements manganese, zinc and copper which are necessary for the anti-oxidant enzymes to function properly - because all these anti-oxidant defence systems work together.

This sort of combination will undoubtedly help to prevent LDL oxidation in the plasma and slow the migration of oxidized cholesterol from the plasma into the artery walls.
In anyone over the age of 20, however, some *oxidized L*DL cholesterol is already inside the artery walls, and here we need different anti-oxidants to stop the disease process.

At this stage the flavonoids come into their own: these anti-oxidants are able to get into the arterial walls, and slow or stop the progression of atheroma there (see Chapter 6, Flavonoids & isoflavones). Beta carotene may be important too: it does not protect circulating LIDL from oxidation, but it prevents cells in the blood vessels' walls from further oxidizing the LDL that is already there.
The evidence that anti-oxidants reduce the risk of coronary artery disease is overwhelming. Conversely, some eminent scientists now say that a poor anti-oxidant status is a better predictor of the risk of heart attacks than high cholesterol levels, blood pressure or any other of the known risk factors.
Oxidative damage also contributes to the late stage complications of diabetes, where the incidence of coronary artery disease, cataract, nephropathy (damage to the kidneys) and neuropathy (nerve damage) scar. The increased oxidative stress in these patients means they have more oxidized lipids in their blood and abnormally low levels of anti-oxidants. Their Vitamin C levels are often so low that some late stage diabetics are close to suffering from scurvy911. High dose anti-oxidants are clearly indicated.

Cataract and age-related macular degeneration (damage to the retina) are the two leading causes of blindness in the developed countries. In the USA, cataract surgery is the single largest item in the Medicare budget, costing some $ 3.2 billion per annum. Worldwide, cataracts blind 50 million people every year; and the tragedy is that much of this is preventable.
The risk of cataract is increased by oxidative stresses such as increased exposure to UV, which oxidises the normally transparent proteins in the lens of the eye, and possibly smoking. The risk is reduced by anti-oxidants such as Vitamins C and E, alpha-lipoic acid, turmeric and possibly beta carotene.

It is estimated that 30-50 per cent of all cases of cataract could be prevented by eating more anti-oxidants. Add a supplement of riboflavin to boost the metabolism of glutathione, an important anti-oxidant in the eye. For comprehensive cover, also add half an aspirin or a spoonful of turmeric, which protect lens proteins from destructive glycosylation reactions (Glycosylation is the cross-linking of proteins.)

Macular degeneration is the other major cause of deteriorating sight. Here again, oxidative damage is involved and anti-oxidants such as E, C and beta carotene are protective. This is probably not the most effective combination of anti-oxidants, however. In primates, the main anti-oxidants in the retina are the carotenoids lutein and zeaxanthin. The optimal strategy to preserve sight, even if it has started to fail, is almost certainly a combination of lutein and zeaxanthin, together with Vitamin C, riboflavin, lycopene, selenium and a turmeric supplement to boost the anti-oxidant enzyme glutathione peroxidase; and the flavonoids in bilberry, a herb traditionally used to treat visual complaints.

In Parkinsonism, toxic tetrahydroquinolones are found in the affected areas of the brain. These compounds are mitochondrial toxins and they cause a decrease in glutathione, an increase in free radical formation, and an increase in nerve membrane damage and death.
If a horse is unlucky enough to eat yellow star thistles, which contain compounds called sesquiterpene lactones, the same sort of things happen - and the horse develops a condition rather like Parkinsonism.
It the changes in mitochandhal function and anti-oxidant status are an important part of the disease process in Parkinsonism, a nutritional approach must be worth trying. This would include a wide spectrum anti-oxidant combination, plus Co-enzyme Q10 and beta carotene to try to improve mitochondrial function; cysteine and alpha-lipoic acid to help boost glutathione levels-, and flavonoids to bind free iron, which 'is released when cells in the brain die and triggers the production of more free radicals.
For flavoncids in this case read hawthorn flavonoids. These powerful anti-oxidants enter the brain (hawthorn is known to cause sedation) and probably enter and protect nerve cell membranes from oxidative damage. I would combine the above with thyme oil, or thymol, which has a similar effect. There is some evidence that nitrous oxide (NO) radicals may be involved, so turmeric, together with beta carotene, or the even more effective carotenoid lycopene, is also worth incorporating in the program.

Finally, add Vitamin E to this multi-component regimen. Vitamin E alone is not very helpful in treating this condition, but chronic Vitamin E depletion is linked to a form of brain damage in animals rather like the damage found in Parkinsonism. In humans who cannot absorb Vitamin E, the risk of Parkinsonism also rises.

Something rather similar to Parkinsonism is found in patients given long-term anti-psychotic medication. They often develop a syndrome, called tardive dyskinesia (TD). Free radical damage is thought to be involved.
Some scientists report that large doses of Vitamin E help, although the evidence is disputed. E is an important antioxidant in the brain, but at least six months of high-dose supplementation are needed to pump up the levels to where they are needed . By that time, much of the nerve damage caused by the anti-psychotic drugs may have already occurred - so it might be better to co-prescribe anti-oxidants from the start.

Cases of asthma are doubling every 10 years. This astonishing trend has generated much research into possible causes of the disease, such as atmospheric pollution, excessive hygiene in childhood, or exposure to the house dust mite - but no case has as yet been proven.
Some work links asthma to car exhaust fumes; diesel fumes in particular have been shown to lead to the formation of dangerous NO (nitrous oxide) radicals and anti-oxidant depletion. This can't be the whole story, however, because although Stockholm has much cleaner air than London, their asthma problem is just as bad.

Nutritional factors have a part to play; recent surveys show that children in the Mediterranean countries are relatively unlikely to get asthma. Decreased anti-oxidant consumption may well make asthmas more likely. Vitamin C is important in protecting the lungs from oxidative damage: a high C intake is linked to better lung function, even in smokers and in patients suffering from chronic obstructive lung disease.
Fish oil reduces inflammation of the airways in high doses (8-10 g/day); but must always be combined with anti-oxidants, preferably Vitamins E and C and flavonoids (see box opposite).

These will reduce the formation of LOPs (Lipid Oxidation Products), which may contribute to the inflammation of the lungs,which underlies asthma.
A high magnesium intake is linked to improved lung function, and some clinicians have found magnesium aerosols useful in relaxing the airways of their asthmatic patients.

Low anti-oxidant consumption is a risk factor for developing arthritis⁽⁵⁴⁾. Some anti-oxidants such as beta carotene reduce symptoms in animal models of arthritis. Other anti-oxidants, such as those found in ginger, reduce joint swelling and pain in clinical trials, but these flavonoid compounds have specific anti-inflammatory properties. (See also Chapter 10, Amino sugars)

Tumour necrosis factor alpha (TNF-alpha) is important in inflammatory conditions such as asthma, Crohn's disease and arthritis, and anti-TNF antibodies have been used with some success in clinical trials of arthritis. The success, however, was tempered; patients who made antibodies to the anti-TNF antibodies developed allergy-type responses.
The nutritional approach may offer a more durable solution. The spice turmeric contains curcumin, a powerful inhibitor of TNF-alpha. In my experience, a combination of turmeric and ginger, which blocks the key inflammatory enzymes, has powerful anti-arthritic properties; when combined with high dose (ie 8-10 g a day) fish or hemp oil, plus 1200 mg per day of Vitamin E and 1-2 g of Vitamin C.

Free radicals are involved in the destruction of tissue that leads to ulcers of the skin and digestive tract. Smoking, which decreases our anti-oxidant defences, is a risk factor-, conversely anti-oxidants can treat these conditions and, if used prophylactically, may prevent them. The flavonoids may be particularly useful here.

The flavonoids are one of the most important groups of compounds derived from plants, Over 20,000 have now been identified since the early days when they were all lumped together as Vitamin P by the great Hungarian biochemist Szent Gyorgyi in 1936 (who also discovered Vitamin C).
Following Szent Gyorgyi's discovery, pharmaceutical companies brought out a range of medicines containing Vitamin P but, by the '60s, most had disappeared. As a natural compound, P could not be patented and because nobody knew exactly what P was, how best to measure it, or even whether P was a single compound or a group, the drug companies found it difficult to produce a reliable product.

Modern techniques have resolved these problems. The flavonoids have been identified and divided into approximately 12 sub-types, many of which have profound anti-oxidant activity.

Each of these groups is under intensive study. Interestingly enough, the results often mirror long-established folk medicine. For example, it has been discovered that one group of flavonoids especially good at mopping up damaging free radicals in the liver is found in particularly high levels in milk thistle, a herb traditionally used to treat liver disease.

Another type of flavanoid which quenches free radicals in the lining of arteries occurs in very high concentrations in hawthorn and yarrow, two plants which have long been used to treat cardiovascular disease.

These are not coincidences. Not all herbal remedies are effective but the tradition goes back thousands of years and is based on generations of experience. Very often, the herbal lore in different countries and even different continents turns out to rely on the same herbs and plants to treat the same diseases, and this again is no coincidence.
So it is not entirely surprising that when the tools of modern science are brought to bear on traditional herbal remedies, they uncover a wealth of valid medical information - information which is forming the basis for new, rational and often highly effective forms of preventative and curative medicine.

The key to the role of flavonoids is that many of them are extremely potent anti-oxidants and anti-flammatory agents.
Different flavonoids work in different tissues of the body; some can enter the brain, for instance, whereas others appear to concentrate in the lining of blood vessels. This means that different flavonoids can be used to target different tissues. For example, a flavonoid which is taken up by the lining of capillaries might be expected to be good for capillary function - and the ginkgo flavonoids, widely used to improve blood flow to the brain, hands and feet, do just this.
Many flavonoids neutralize free radicals, including the highly dangerous hydroxyl radical. One group is particularly good at quenching the radicals which cause liver damage (these are the flavonoids found in milk thistle).

### Phytate - can act as an anti-oxidant

The second anti-cancer ingredient is phytate, generally regarded as another anti-nutrient because it binds iron. But in certain conditions this is a good thing, as excess (free) iron in the body is a potent source of free radicals, and a potential carcinogen.
When phytate binds iron, it is effectively acting as an antioxidant. This helps to explain why phytate is a powerful inhibitor of colon cancer, where free iron is one of the key causative factors. (I don't recommend phytate for everyone, however. Iron deficiency anaemia is still widespread, particularly among women of child-bearing age, and too much phytate could worsen an existing anaemia.)

### Phytosterols - protect against carcinogenic bile acids

Third up are the phytosterols, the plant equivalents of cholesterol. They are poorly absorbed and remain in the gut, where they are thought to protect against the harmful effects of certain (secondary) bile acids. These bile acids are formed from cholesterol and have mutagenic and carcinogenic properties. This may be why some phytosterols are capable of reducing the incidence of colon cancer by as much as 50 per cent.

### Saponins - anti-mutation, anti-oxidants

Fourth on the list are the saponins, anti-oxidants which protect against free radical damage. Lab tests have shown that saponins prevent mutations that can lead to cancer.

### Phenolic compounds - protect DNA

Fifth are a group of phenolic compounds. These too possess anti-oxidant activity, and are thought to protect DNA from attack by certain categories of carcinogen.

Isaflavones block oestrogen, a hormone linked to an increased risk of breast and other hormone-dependent cancers. They act rather like Tamoxifen, a drug widely used to treat and prevent breast cancer.

Prostate cancer, like breast cancer, is usually hormone-dependent. But whereas breast cancer is encouraged by oestrogen, prostate cancer is often driven by testosterone. Isoflavones which block testosterone reduce the tendency for prostate cancers to grow.

Some flavonoids bind to dangerous free iron and copper in the body, thereby stopping free radical formation. Many are capable of locking up free oxygen and preventing the oxidation of ascorbic acid, thereby protecting Vitamin C in fruit, fruit juices, and in the body.

One flavonoid, quercitin, found in onions and apples may be one of the most cardio-protective substances yet discovered.
The Zutphen Elderly Study measured the flavonoid content of the diet of their subjects and discovered that the number of cardiac deaths in the group eating the most flavonoids was a quarter of the death rate in the group which ate the least flavonoids. And quercitin accounted for two-thirds of the total flavonoid intake.

In fact, all mortality rates were lower in the high flavonoid group, even when other dietary anti-oxidants such as Vitamins C and E were ruled out.

Since Zutphen, two more trials have found that quercitin (a powerful anti-oxidant and anti-inflammatory agent is cardio-protective.
There was some controversy over whether quercitin is absorbed from the gut, because little is found in the bloodstream. Recent work shows that quercitin and related compounds are absorbed and concentrated elsewhere in the body.
There's other evidence that flavonoids are absorbed. Tannins, for example, which consist of long chains of flavanoids linked together, protect against stroke in hypertensive animals. Tannins are found in, e.g. tea, wine, quince and persimmons.
Or try eating a good helping of beetroot. The purple pigment in beets consists of flavonoids which, when eaten, are absorbed from the gut and excreted in the urine - a colorful fact you can check for yourself. Combine beets with rhubarb or spinach at the same meal to get the most spectacular results.

Procyanidins are an extremely promising group of flavonoids. They are well absorbed from the gut, and are already used (in the form of pycnogenol and grapeseed extracts) to treat arthritic conditions, because of their ability to quench free radicals and stop the breakdown of synovial (lubricating) fluid inside inflamed joints.

These flavonoids also target blood vessels and, once there, protect the connective tissue in the artery walls by exerting a powerful anti-oxidant, anti-inflammatory and anti-permeability effect. They also block enzymes which have a destructive effect on the connective tissues.

These protective actions mean the high procyanidin content of black grapes (and red wine) is probably one of the main factors underlying the so-called French paradox. The French eat a high fat diet, yet are relatively immune from heart disease. There are probably several factors involved, including the widespread use of olive oil, but there is evidence that consumption of two to four glasses of red wine a day reduces the risk of a heart attack by an astonishing 40 per cent.

Anyone seriously concerned about reducing their risk of heart attacks should consider combining quercitin with a procyanidin product, e.g. grapeseed extract.
The two types of flavonoid, though similar, appear to block two distinct steps in the chain of events that leads to atheroma formation. Quercitin's main role is to protect lipids in the blood from oxidising; whereas the procyanidin's main role is to prevent oxidative damage from occurring in the blood vessel wall.
The procyanidins' ability to bind to and protect the fibres in the vessel walls (like collagen and elastin) from oxidative or enzyme attack helps to reduce the amount of damage to the walls. If there is already damage, the two flavonoids seem to stop the site becoming inflamed and slow the furring of the arteries.

Procyanidins are best used preventatively, but even after a heart attack they can help. Their phenomenal ability to scavenge free radicals means that a procyanidin product will reduce your risk of developing a life-threatening arrhythmia after the attack.

Because of the ability of procyanidins to keep blood vessels healthy, it's not surprising that reports have appeared describing improvements in varicose veins, oedema and haemorrhoids.

Anyone suffering from any of these should try a procyanidin product for at least two months, although in some cases improvement is noticeable in one month. Vitamin C should be taken with it, and in serious cases an additional glucosarnine supplement.

Another important cause of sight loss in diabetics is the growth of new blood vessels supplying the retina. The procyanidins block this effect, another reason why they are helpful in this condition. Other flavonoids can help diabetics too. The cholesterol in the blood is more prone to oxidation than in non-diabetics, which is one reason why diabetics suffer more heart attacks. In a recent study, a flavonoid preparation (Diesmin) not only reduced the rate of cholesterol and lipid oxidation but also reduced the rate that proteins were damaged (cross-linked) by the excess blood sugar. This would not only reduce blood vessel damage in the eye and elsewhere in the body, but also protect against cataracts and renal damage, where excess cross-linking is involved.

Procyanidins are being incorporated into the latest cosmetics. They form a Protective shield around the collagen fibres which give skin its firmness and texture, and Protect them against the enzymes which break down these fibres, and against free radical damage too.
It's early days, but Procyanidin's anti-allergic, anti-inflammatory and anti-oxidant Properties could constitute a major cosmetic break-through, especially when combined with other anti-aging nutrients such as the amino sugars.
Procyanidins target the bacteria which cause dental decay. The flavonoids seem to stop the bugs sticking to the teeth and to dental plaque. Some dental scientists are looking at these flavonoids as a way Of slowing down tooth decay. The anti-inflammatory effects may also help to control or minimize gingivitis (gum disease) which is responsible for more tooth loss than dental decay.

Flavonoids boast an extensive array of anti-cancer effects which add up to an extremely impressive cancer defence package:
Free radicals damage DNA. Many flavonoids are potent anti-oxidants which mop up large numbers of free radicals, and reduce the amount of DNA 'hits'. Free radicals also damage cell membranes. This damage might also lead to tumor formation, so once again anti-oxidants, like flavonoids, should help.
Not surprisingly, a great deal of nutritional research is concentrating on just this area.
Some of the most active and best researched compounds include:
   quercitin - onions
   ellagic acid - walnuts, pecan nuts
   caffeic acid - coffee beans
   chlorogenic acid - tomatoes
   epigallocatechingallate - tea
   carnosic acid - rosemary
   genistein - soy.

Here are the six anti-cancer elements in say beans.

Protease inhibitors (lectins) - which block genes which promote cancer.
Soy beans contain growth-inhibiting substances called protease inhibitors, which act to reduce the spread of cancer.
Recent work has demonstrated that protease inhibitors also block the action of a number of genes which cause cancer. Most protease inhibitors are destroyed by cooking, but there is evidence that enough survive to confer a significant protective effect.

King of the isoflavones, and subject of well over 300 research papers to date, is genistein. Genistein has little effect on normal cells - but it is a powerful inhibitor of nearly every cancer cell type examined so far.

Its anti-cancer effects are wide because its mode of action is so profound: it inhibits several of the products of oncogenes, genes which cause cancer.

Soy protects against heart disease as well.
One of the most insidious aspects of coronary artery disease (CAD) is that it is a hidden disease. For most people, the first sign that anything is wrong is the first heart attack. The great majority of survivors are left with a permanently damaged heart and a long-term risk of complications.
Even advanced cases of CAD are often not diagnosed in time. An American investigation called the Sudden Death Study, discovered that an astonishing one in four people who died suddenly of a heart attack had seen their doctor in the week before they died. But they had not been diagnosed accurately, and had not been hospitalized. This is why with coronary artery disease, as in so many other diseases, prevention is better than cure.
Diet is the key. A diet rich in animal fats and low in anti-oxidants and fish oil is a fast route to a heart attack, as is smoking. And so is high blood cholesterol.

The large bowel, which is where the majority of gastrointestinal cancers occur, is full of four to five hundred different species of bacteria, known in medical language as 'flora'. Some of these can cause serious illness, while others are associated with positive health.

Ever since the beginning of this century, doctors have experimented with different diets in an attempt to modify the gastrointestinal flora, and push it in a 'healthy' direction (without much success).

There are at least two types of health-promoting bacteria, the lactobacilli and the bifidobacteria. Some of these are found in live yogurt, and various scientists and nutritionists have used yogurt to try to change the flora of the lower bowel.
However, the bacteria have a limited shelf-life, even when freeze-dried, and many of them are unable to survive the acid conditions in the stomach. Even if the bacteria do arrive in the colon, they have to compete with the dense population of hostile bacteria that are already there.

As long as you eat a daily helping of live yogurt, some lactobacilli and bifidobacteria remain in the gut, but they disappear almost immediately when the yogurt diet stops. Prebiotics have none of these disadvantages. They are stable, safe (they are found in many staple foods), and they have a longer-lasting effect on the gut's flora. They encourage the growth of 'healthy' bacteria, and put a check on other bacteria which can cause disease by overgrowth or by producing toxins.
We'll consider two of the main types of natural pre-biotic -inulin and oligo-fructose. The general rule is that the fresher the vegetable, the higher its inulin content. When plants such as onions are stored for long periods of time, and particularly in cold or cool storage, their pre-biotic content declines dramatically.
Because most of us buy our fruit and vegetables from supermarkets where the foods may have been in cold storage for months, this could mean that your pre-biotic intake is actually very low.

A low intake of pre-biotics leads to increased numbers of disease-causing bacteria in the gut - which could be the cause of many gastrointestinal and other health complaints.

Unlike most sugars and starches, pre-biotics cannot be digested and they pass into the colon intact. Once there, they act as a growth enhancer for the health-promoting lactobacilli and bifidobacteria.

As the 'good' bacteria multiply, they secrete enzymes which break down pre-bictics into acids such as acetic and butyric acid. These inhibit the growth of disease-causing bacteria. The 'good' bacteria also secrete antibiotic substances which restrain the 'unhealthy' bugs, including most of those responsible for food poisoning.
As a result, the balance of the flora of the gut tips in a healthy direction. The flourishing lactobacilli and bificlobacteria in the gut join gastric acid, the digestive enzymes and the immune system in 'crowding' out disease-causing bacteria. You'll benefit from improved intestinal 'regularity' and an increased resistance to food poisoning the gut of breast-fed infants, but a mere 25 per cent in bottle-fed infants. This explains why breast-fed babies are more resistant to stomach upsets and diarrhoea.
Recent studies show that live yogurt cultures fed to infants significantly reduce their risk of contracting diarrhoea, and speed recovery it given as a treatment for diarrhoea.
As we age, the proportion of bifidobacteria and lactobacilli gradually falls. This is one reason why we become more prone to gastrointestinal upsets and it is probably also linked to the agerelated increase in the risk of bowel cancer and other illnesses'.

The risk of colon cancer is increased by a diet high in animal fats - a diet which causes more bile to be secreted. Unhealthy gut bacteria convert bile acids into cancer-causing compounds which increase the risk of liver cancer.

Pre-biotics reduce the disease-causing bacteria in the gut, and the amount of cancer-causing compounds they produce which could well have a protective effect. Finally, as the bifids grow they bind free iron, thereby reducing levels of free radicals in the colon. This must be another cancer-preventing property.

Read the contents' list on a carton of yogurt and you'll see it contains significant amounts of thiamin, riboflavin and other vitamins. This is because the lactobacilli and bifidobacteria make B vitamins, and are probably the major species of bacteria in the colon which do this.

B depletion is surprisingly common in the developed countries, and low B levels are a major risk factor for coronary artery disease. So pre-biotics, by increasing the good bacteria in the gut and B vitamin levels, will be cardioprotective by lowering homocysteine, and simultaneously raising HDL levels.

This is one way in which cats and inulin contribute to a healthy heart; although LDL cholesterol reduction also plays a role.

Short chain pre-biotics (ie FOS) are rapidly fermented, stimulating the production of bifidobacteria (bifidogenesis) in the proximal colon. As they grow they bind the bile acids present in this part of the gut and remove them from the body. This lowers LDL cholesterol levels and confers additional cardio-protection. The combination of bifidogenesis and bile acid binding is also likely to be cancer protective, especially if FOS is combined with longer chain pre-biotics.

The edible fats and oils, known collectively as fatty acids, are basically similar compounds. Oils, however, melt at lower temperatures than fats, and at room temperature oils are liquid and fats are solid.
Fatty acids (fats and oils) are a rich source of calories, which can either be 'burned' to produce energy, or stored as fat for lean times ahead. They are also incorporated into cell membranes and other tissues, where they have an important structural role.

Finally, fatty acids are metabolised into compounds called eicasanoids. Fats and oils produce quite different eicosanoids: broadly speaking, fats form eicosanoids which increase inflammation, and oils produce eicosanoids which reduce inflammation.

This difference is important to know, because many chronic diseases are basically inflammatory conditions. These include arthritis (inflammation of the joints), eczema (inflammation of the skin), asthma (inflammation of the lungs) and coronary artery disease (inflammation of the arteries).

Your risk of these conditions is affected by your genetic makeup, tobacco consumption and the amount of anti-oxidants in your diet: but the fats and oils we consume are also important.

Saturated fats raise the levels of 'bad', ie LDL, cholesterol in the blood. Fish oil, on the other hand, slows the formation of LDL cholesterol, reducing the risk of heart attacks.

The medical profession found it hard to believe that a simple switch from fats to oils could have significant health benefits, but the sheer weight of all the clinical trials which have shown positive health benefits has begun to change their opinion. The evidence is particularly good in the area of coronary artery disease,- and the PUFAs are rapidly gaining a role in even conventional circles.

Not all PUFAs (poly- unsaturated tatty acids) are the same. Some of them are more important than others, and a few are so important they are termed, collectively, the Essential PUFAs.

There are two families of essential pc iy-unsatu rated fatty acids, Omega 6 and Omega 3, both of which are oils. These oils are vital for the functioning of every cell in our bodies, and yet our bodies cannot make them. We have to obtain them from our diet, and in that sense they are similar to vitamins.
Once the oils have been absorbed from our food, our enzymes make all the other Omega 3 and 6 PUFAs our cells and systems need.

PUFAs are a powerful force for good health but, as with other powerful agents, they should not be taken indiscdminately. The ratio of the various PUFAs in the diet is important, and the fact that in most mammals' cells the level of Omega 6 (from vegetable sources) is three to tour times higher than the Omega 3 content (from fish sources), gives us a painter as to what we should aim for.

The problem with poly-unsaturated tatty acids (PUFAs) is that they are very prone to going rancid, or oxidising. It's fine to eat a high PUFA diet it the PUFAs are in unprocessed foods such as nuts and grains, because these foods contain their own antioxidants, such as Vitamin E, carotenoids and flavonoids. Without these they would go rancid, and the seeds, grains and nuts would not survive long enough to propagate the species. But if your intake of PUFAs is in the form of refined poly-unsaturated oils and spreads, you could be in trouble.

These processed foods have had the naturally occurring antioxidants stripped away, and are therefore highly prone to being oxidized. This has two potentially very serious effects.

Firstly, the PUFAs form lipid oxidation products (LOPs). LOPs are extremely toxic: they literally rip holes in the lining of the arteries and are therefore a substantial risk factor for heart disease. Secondly, as the PUFAs oxidize they soak up antioxidants in the body, leaving an excess of free radicals and causing accelerated aging.

The end result is an increase in the risk and severity of chronic degenerative diseases from heart disease to cancer to asthma.

At the Royal Prince Alfred Hospital in Sydney, Australia, epidemiologists have linked the huge increase in childhood asthma with a five-fold increase in margarine consumption since the War. Their theory. is that the increased PUFA content in the diet has led to an increase in inflammatory and toxic LOPs, which leave the airways raw, and trigger the asthma.

Because poly-unsaturated oils are so prone to oxidation, it is very important to combine them with an anti-oxidant preparation, especially if you smoke.

Otherwise, you are likely to increase the load at PUFA radicals and oxidation products in your body, which are actually harmful to the arteries. This may explain the disappointing results of a recent trial which found that fish oil supplements on their own have little effect on preventing atheroma formation.
The ideal anti-oxidant combination should contain
- Vitamin E (400 IU/day) - Mixed flavonoids (100-500 mg)
- Vitamin C (500-1 000 mg) - Co-enzyme Q10 (30-120 mg/day)
- Mixed; carotenoids (10-20 mg).

Co-enzyme Q 10 is particularly recommended. Apart from its other benefits (see Chapter 9, Co-enzyme Q I0), it is very good at preventing the increase in free radicals otherwise caused by fish oil supplements.

Co-enzyme Q10 is often referred to as Vitamin Q, but although it is vital to life, and occurs in trace amounts in certain foods such as sardines, Q10 is not technically a vitamin because we can produce small amounts of it ourselves in the liver.

Unfortunately, the process requires at least six other vitamins and minerals - and most people are depleted in one or more of these. Heavy drinking and liver disease slow the making of Q10 even further. The final problem is that after the age of 40 or so, our ability to make Q10 declines, and levels of Q10 in the diet are too low to compensate for this.

Amino sugars are among the most important building blocks in the body.

Dr. Frances Burton, an expert in amino sugars at the University of British Columbia, expresses it beautifully: "Amino sugars make up the structure of all tissues, an the surface of cells and in the spaces in between them; forming the substance which binds cells together, the membranes which envelop them and the protective layers which cover them."

Macro-molecules built up from amino sugars, called GAGs and PGs, together with the proteins called collagen and elastin, make up the framework for all our tissues.

Combined in different proportions, they make tissues soft, slippery, squashy, stretchy or strong. They give our bodies shape, organization, definition and function.

GAGs and collagen make up tendons, ligaments, heart valves, skin and finger-nails. Combined with another protein, elastin, they make cartilage in joints and the discs in the spine.

All of these macro-molecules are constantly being broken down and replaced as part of the body's on-going general maintenance programs.

How fast this happens is crucial in maintaining the strength and elasticity of every tissue in the body. However, there is one major problem.

Based an our current understanding of arthritis, a nutritional programs which combines glucosamine with natural antiinflammatory flavonoids (see Chapter 6, Flavonoids & isaflavones) should greatly reduce the symptoms and the risk of the disease.

This is likely to produce the best results in the elderly, who are more depleted in glucosamine and anti-oxidants, such as flavanoids. It's a combination which has not yet been properly tested, but it seems logical that such a nutritional programs could halt, or even reverse the progress of arthritis.

The cells lining the gut have a very high turnover rate. In chronic inflammatory conditions, the rate is even faster.

In these conditions, the rate of cell growth may outstrip the rate of glucosamine and GAG (glycosaminoglycan) production. In fact, the inflammation itself may inhibit the making of GAGs, and increase the rate at which they are broken down.

Patients with active inflammatory bowel disease (including Crohn's Disease and Ulcerative Colitis) have very low levels of GAGs in their intestinal walls. A depletion here would be expected to cause local vascular problems, increasing leakage of fluid into the surrounding tissues, and contribute to several distinct types of local tissue damage that are, in fact, all found in chronic inflammatory bowel disease.

Low levels of amino sugar compounds bring another set of problems. They would eventually affect the thin but vital glycolipid layer which protects the intestinal wall.
Since the gut, more than any other organ, is constantly challenged by bacteria, viruses, digestive juices and dietary antigens, losing this vital protection could lead to health problems, including food allergies, which are thought to occur in conditions where the gut wall is abnormally permeable.

In the exposed skin of the face and hands, much of the aging is caused by free radicals liberated by sunlight.
Free radicals damage collagen and elastin fibres in the skin, and the GAGs and PGs. This damage to the extra-cellular matrix leads to a loss of firmness, plumpness and elasticity, and is a large part of skin aging.
The amino sugar compounds in the skin are constantly being broken down and replaced. As much as one fifth of the glucose in the blood is destined for connective tissue formation. But if the glucosamine-producing enzymes slow down, as they do with age, they cannot keep pace with the deterioration caused by exposure to ultra-violet light (UV), cigarette smoke, pollution and other sources of free radicals.

The connective tissue that gives the skin strength, elasticity and firmness deteriorates, with all-too-obvious results.

To protect the skin, you need amino sugars such as glucosamine, and Vitamin C and zinc for collagen and elastin synthesis. To maintain the exta-celfular matrix requires an antioxidant mix containing the procyanidin flavonoids (e.g. bilberry or grapeseed), which concentrate in the connective micro-fibres and protect them from free radical damage; plus mixed carotenoids, which have a similar effect.

You also need an anti-glycosylant. Glycosylation (the attachment of sugar molecules) of collagen and elastin increases with aging. This disrupts the connective tissue in a process known as cross-linking. This has the unfortunate effect of leaving the skin less elastic, less permeable and more prone to wrinkles.
Half a tablet of aspirin helps prevent glycosylation, as does a tablespoon of turmeric. Vitamin C has a similar effect, and is another essential part of the anti-ageing programme; especially as it is essential for the synthesis of the skin protein collagen.
A supplement of silicic acid may also be appropriate. High levels of aluminium damage the fibroblasts and other cells responsible for building and repairing the extra-ceifular matrix, in the skin and elsewhere. Silicic acid is the most effective shield against ingested aluminium, and can enhance the regeneration of the extra-ceflular matrix.

Methyl groups, like vitamins, are essential in our diet. Foods that contain significant levels of the methyl groups are, in descending order, sugar beet, sugar cane, prawns, shrimps and eggs.
To give an idea of the importance of methyl groups, the nervous system, the immune system, the heart and blood vessels, the kidneys and the liver all depend on methyl groups to function normally.

A diet low in methyl groups damages all the above systems. Stress becomes more destructive, toxins become more toxic, carcinogens more carcinogenic.
In fact, a lack of methyl groups in the diet is the only dietary deficiency known to be directly carcinogenic. If there are not enough methyl groups, DNA reproduction can go wrong, leading to the activation of ancogenes (cancer-causing genes).
To appreciate why a deficiency of methyl groups in the diet is so dangerous, we need to understand a process called the methyl group cycle (see diagram an next page). It's also important to know that excessive levels of the amino acid homocysteine in the body are a major risk factor for heart disease and Alzheimer's (see Chapters 14, Heart disease and 17, A healthy brain).
Methyl groups are a simple combination of carbon and hydrogen atoms. You will see from the diagram that methyl groups from the diet combine with homacysteine in the body to form methionine. Methionine is then turned into S-adenosyl methionine (SAM).

SAM passes an the methyl groups in the body to produce many essential compounds. These include creatine and carnitine (important in energy production), phospholipids, (essential molecules involved in-cell membrane and especially nerve health), RNA and DNA, the stress hormones epinephrine and nor-epinephrine, and the neurotransmitters involved in mood. Methyl groups are also essential to the basic functioning of the immune system.

If there are inadequate methyl groups in the diet, all these functions are impaired. But there is a further serious implication of insufficient methyl groups in the diet.

After SAM has donated its methyl group it becomes Sadenosyl homocysteine, which breaks down into the toxic amino acid homocysteine.

If there are too few methyl groups from the diet to transform this hamacysteine back again into methionine, levels of hornocysteine rise and so therefore does the risk of cardiovascular disease and Alzheimer's disease.

The body cannot synthesize methyl groups, and therefore a constant dietary intake of methyl groups is essential to maintain the cycle, in order to keep levels of SAM up and levels of homocysteine down.

The principal dietary sources of methyl groups are, in descending order, the nutrients betaine,-chaiine and methionine; and to a lesser extent the Vitamins B6, B 12 and folic acid. The vitamins are not the best donors; betaine is far more effective.

Under conditions of stress (such as disease), the need for methyl groups increases. This is because methyl groups are needed for the formation of stress hormones, for various defence mechanisms and for the synthesis of polyamines, RNA and DNA, all of which are needed for tissue repair.

When stress increases the demands for methyl groups, the resulting shortfall in methyl groups inevitably leads to an increase in homocysteine - another reason why stress is bad for your health.
As an excellent methyl group donor, betaine is very effective at lowering levels of homacysteine. Most humans, however, do not consume much betaine; and in this situation, the B vitamins become the next line of defence. One recent study found that people who ate high levels of folic acid (a B vitamin) were 69% less at risk of a fatal heart attack than those whose diet contained low levels of folic acid.

Sadly, B vitamin deficiency is also ail too common - and this explains why excessive levels of homocysteine (and therefore heart disease and Alzheimer's disease) are so common.

A strong B complex preparation reduces levels of homocysteine. This is why supplements of folic acid and Vitamins B6 and B12 are increasingly being used to reduce homocysteine levels, and the risk of homocysteine-related cardiovascular and neurological diseases.

There are several mixtures containing vitamines, minerals and other substances useful as food supplements known to the state of the art which exhibit health promoting effects. These mixtures contain usually substances in a not specified amounts thereby causing possibly unwanted side effects. Some of these mixtures are used to effect health in a general manner. Such mixtures are considered to produce less efficacy in case specific symptoms of a disease need to be improved. Therefore the problem of the present invention is deemed to consist in providing defined mixtures of compounds useful as food supplements to trigger specific symptoms of selected diseases.

The mixtures of compounds as provided by the invention exhibits different favourable effects with respect to several important aspects of human health as mental decline, diabetes, arthritis, osteoporosis, asthma and age related eye disease in particular macular degeneration. These mixtures may be useful as food additives or as medicaments.
The effects of nutraceutical intervention are always filtered through the nutritional baseline of the subject or by other lifestyle factors such as smoking and activity levels or by genetic variations. One cannot change genetic variations. But in order to achieve optimal results the neutraceutical intervention should be combined with a reduction or cessation in smoking, a reduction in sodium intake and/or an increase in activity levels.

The invention refers to several mixtures of compounds comprising one or several of the following compounds but avoiding to contain all of them in one mixture:
a) 800 mcg (2664IU) of Vitamin A, 500 mg of Vitamin C, 15 mcg of Vitamin D, 200IU of Vitamin E, 200 mcg of Vitamin K,
b) 10 mg of Beta carotene, 6 mg of Lutein, 5 mg of Lycopene, 100 mcg of Zeaxanthin,
c) 7.5 mg of Vitamin B1, 7.5 mg of Vitamin B2, 15 mg of Niacin, 15 mg of Pantothenic acid, 7.5 mg of Vitamin B6, 200 mcg of Folic acid, 6.75 mcg of Vitamin B12,
d) 200 mcg of Selenium (preferably in the yeast form), 10 mg of Zinc, 100 mg of Calcium, 50 mg of Magnesium,120 mcg of Chromium, 2 mg of Copper, 4 mg of Manganese, 100 mcg of Iodine, 100 mcg of Molybdenum,
e) 200 mcg of Biotin, 1000 mg of Betaine, 250 mg of Oligoproanthocyanidins (OPC), 150 mg of Polyphenol complex, 40 mg of Isofloavones in particular genistein and/or daidzein , 600 mg of Omega 3, 4 to 6 g of Oligosaccharides (FOS) in particular oligo-fructose and/or beta glucan, 4 to 6 g of inulin, 30-60 mg of Co-Q10,
f) 1000 mg of Glucosamine possibly additional substances for the purpose of stabilisation and formulation of the above mentioned compounds of this claim.

The amount given as abovementioned to determine the quantity of each single compound within the master mixture defines the average content of this said compound. This content may vary within a upper or lower limit of up to 15% of this value caused for example by varations within the compound sources or methodlogical variatons from weighing or packaging Likewise in a preferred embodiment of the invention the amount of each single compound may fall within the upper and lower limits as defined by table 1 as far as limits have been provided by this table.

The invention refers to a Mixture of compounds comprising at least
a) 800 mcg (2664IU) of Vitamin A, 1000 mg of Vitamin C, 400IU of Vitamin E, 200 mcg of Vitamin K,
b) 10 mg of Beta carotene, 6 mg of Lutein, 5 mg of Lycopene, 100 mcg of Zeaxanthin,
c) 7.5 mg of Vitamin B1, 7.5 mg of Vitamin B2, 15 mg of Niacin, 15 mg of Pantothenic acid, 7.5 mg of Vitamin B6, 200 mcg of Folic acid, 6.75 mcg of Vitamin B12,
d) 200 mcg of Selenium (preferrably in the yeast form), 10 mg of Zinc, 120 mcg of Chromium, 2 mg of Copper, 4 mg of Manganese, 100 mcg of Iodine, 100 mcg of Molybdenum,
e) 200 mcg of Biotin, 1000 mg of Betaine, 250 mg of Oligoproanthocyanidins (OPC), 150 mg of Polyphenol complex, 1000mg of alpha lipoic acid, 600 to 5000 mg of mixed Omega 3 and 6, 4 to 6 g of Oligosaccharides (FOS) in particular oligo-fructose and/or beta glucan, 4 to 6 g of inulin
and possibly additional substances for the purpose of stabilisation and formulation of the above mentioned compounds of this claim.

The invention refers further to preparation of this mixture according to the immediately aforegoing section by first producing the said single compounds by chemical synthesis or isolating them from natural sources, therafter putting the single compunds together in a suitable vessel in relative amounts as to end up with the specified amount for each compund, then mixing the compounds after having put them together as aforementioned, and adding additional substances for stabilisation and/or formulation of this mixture.
The invention refers also to use of this same mixture for preparation of a food additive with beneficial effects preventing, curing, or improving of symptoms of diabetes and to use of this same mixture for preparation of a medicament for preventing, improving, or curing of diabetes.

The invention refers to a mixture of compounds comprising at least
a) 800 mcg (2664IU) of Vitamin A, 500 mg of Vitamin C, 200IU of Vitamin E, 200 mcg of Vitamin K,
b) 10 mg of Beta carotene, 6 mg of Lutein, 5 mg of Lycopene, 100 mcg of Zeaxanthin,
c) 7.5 mg of Vitamin B1, 7.5 mg of Vitamin B2, 15 mg of Niacin, 15 mg of Pantothenic acid, 7.5 mg of Vitamin B6, 200 mcg of Folic acid, 6.75 mcg of Vitamin B12,
d) 200 mcg of Selenium (preferably in the yeast form), 10 mg of Zinc, 120 mcg of Chromium, 2 mg of Copper, 4 mg of Manganese, 100 mcg of Iodine, 100 mcg of Molybdenum,
e) 200 mcg of Biotin, 5000 mg of Oligoproanthocyanidins (OPC), 150 mg Of Polyphenol complex, 600 mg of Omega 3, 100 mg of Co-Q10
f) 1000 mg of Glucosamine
and possibly additional substances for the purpose of stabilisation and formulation of the above mentioned compounds of this claim.

The invention refers further to reparation of this mixture according to the immediately aforegoing section by first producing the said single compounds by chemical synthesis or isolating them from natural sources, therafter putting the single compunds together in a suitable vessel in relative amounts as to end up with the specified amount for each compund, then mixing the compounds after having put them together as aforementioned, and adding additional substances for stabilisation and/or formulation of this mixture.
The invention refers also to use of this same mixture as food additive with beneficial effects on preventing, curing, or improving of symptoms of osteo-arthritis as well as to use of this mixture for preparation of a medicament for preventing, improving, or curing of osteo-arthritis.

The invention refers to a mixture of compounds comprising at least
a) 800 mcg (2664IU) of Vitamin A, 500 mg of Vitamin C, 20 mcg of Vitamin D, 200 mcg of Vitamin K,
b) 10 mg of Beta carotene, 6 mg of Lutein, 5 mg of Lycopene, 100 mcg of Zeaxanthin,
c) 7.5 mg of Vitamin B1, 7.5 mg of Vitamin B2, 15 mg of Niacin, 15 mg of Pantothenic acid, 7.5 mg of Vitamin B6, 200 mcg of Folic acid, 6.75 mcg of Vitamin B12,
d) 200 mcg of Selenium (preferably in the yeast form), 10 mg of Zinc, 100 mg of Calcium, 50 mg of Magnesium,120 mcg of Chromium, 2 mg of Copper, 4 mg of Manganese, 100 mcg of Iodine, 100 mcg of Molybdenum,
e) 200 mcg of Biotin, 250 mg of Oligoproanthocyanidins (OPC), 150 mg of Polyphenol complex, 40 mg of Isofloavones in particular genistein and/or daidzein, 100 mg of Co-Q10,
f) 1000 mg of Glucosamine
and possibly additional substances for the purpose of stabilisation and formulation of the above mentioned compounds of this claim.

The invention refers further to preparation of a mixture according to the immediately aforegoing section by first producing the said single compounds by chemical synthesis or isolating them from natural sources, therafter putting the single compunds together in a suitable vessel in relative amounts as to end up with the specified amount for each compund, then mixing the compounds after having put them together as aforementioned, and adding additional substances for stabilisation and/or formulation of this mixture.

The invention refers also to use of this same mixture for preparation of a food additive with beneficial effects for preventing, curing, or improving of symptoms of osteoporosis as well as of use of this same mixture for preparation of a medicament for preventing, improving, or curing of osteoporosis.

The invention refers to a mixture of compounds comprising at least
a) 800 mcg (2664IU) of Vitamin A, 1000 mg of Vitamin C, 15 mcg of Vitamin D, 400IU of Vitamin E, 200 mcg of Vitamin K,
b) 10 mg of Beta carotene, 6 mg of Lutein, 5 mg of Lycopene, 100 mcg of Zeaxanthin,
c) 7.5 mg of Vitamin B1, 7.5 mg of Vitamin B2, 15 mg of Niacin, 15 mg of Pantothenic acid, 7.5 mg of Vitamin B6, 200 mcg of Folic acid, 6.75 mcg of Vitamin B12,
d) 200 mcg of Selenium (preferrably in the yeast form), 10 mg of Zinc, 100 mg of Calcium, 50 mg of Magnesium,120 mcg of Chromium, 2 mg of Copper, 4 mg of Manganese, 100 mcg of Iodine, 100 mcg of Molybdenum,
e) 200 mcg of Biotin, 1000 mg of Betaine, 250 mg of Oligoproanthocyanidins (OPC), 150 mg of Polyphenol complex, 500 mg of curcuminoids, 100 mg of ginko biloba, 600 to 10 000 mg of mixed Omega 3, 60 - 90 mg of Co-Q10,
and possibly additional substances for the purpose of stabilisation and formulation of the above mentioned compounds of this claim.

The invention refers further to preparation of a mixture according to the immediately aforegoing section by first producing the said single compounds by chemical synthesis or isolating them from natural sources, therafter putting the single compunds together in a suitable vessel in relative amounts as to end up with the specified amount for each compund, then mixing the compounds after having put them together as aforementioned, and adding additional substances for stabilisation and/or formulation of this mixture.
The invention refers also to use of the same mixture for preparation of a food additive with beneficial effects on preventing, curing, or improving of symptoms of asthma as well as of use of the same mixture for preparation of a medicament for preventing, improving, or curing of asthma.

The invention refers to a mixture of compounds comprising at least
a) 800 mcg (2664IU) of Vitamin A, 500 mg of Vitamin C, 200IU of Vitamin E, 200 mcg of Vitamin K,
b) 10 mg of Beta carotene, 6 mg of Lutein, 5 mg of Lycopene, 100 mcg of Zeaxanthin,
c) 7.5 mg of Vitamin B1, 7.5 mg of Vitamin B2, 15 mg of Niacin, 15 mg of Pantothenic acid, 7.5 mg of Vitamin B6, 200 mcg of Folic acid, 6.75 mcg of Vitamin B12,
d) 200 mcg of Selenium (preferrably the yeast form), 10 mg of Zinc, 120 mcg of Chromium, 2 mg of Copper, 4 mg of Manganese, 100 mcg of Iodine, 100 mcg of Molybdenum,
e) 200 mcg of Biotin, 1000 mg of Betaine, 250 mg of Oligoproanthocyanidins (OPC), 150 mg of Polyphenol complex, 40 mg of Isofloavones in particular genistein and/or daidzein , 600 mg of Omega 3, 4 to 6 g of Oligosaccharides (FOS) in particular oligo-fructose and/or beta glucan, 4 to 6 g of inulin, 60 - 90 mg of Co-Q10,
and possibly additional substances for the purpose of stabilisation and formulation of the above mentioned compounds of this claim.

The invention refers further to preparation of a mixture according to the immediately aforegoing section by first producing the said single compounds by chemical synthesis or isolating them from natural sources, therafter putting the single compunds together in a suitable vessel in relative amounts as to end up with the specified amount for each compund, then mixing the compounds after having put them together as aforementioned, and adding additional substances for stabilisation and/or formulation of this mixture.
The invention refers also to use of the same mixture of for preparation of a food additive with beneficial effects on preventing, curing, or improving of symptoms of mental decline as well as use of the same mixture for preparation of a medicament for preventing, improving, or curing of mental decline.

The invention refers to a mixture of compounds comprising at least
a) 800 mcg (2664IU) of Vitamin A, 500 mg of Vitamin C, 265 mg (400IU) of Vitamin E, 50 mcg of Vitamin K,
b) 10 mg of Beta carotene, 6 mg of Lutein, 5 mg of Lycopene, 100 mcg of Zeaxanthin,
c) 7.5 mg of Vitamin B1, 7.5 mg of Vitamin B2, 15 mg of Niacin, 15 mg of Pantothenic acid, 7.5 mg of Vitamin B6, 200 mcg of Folic acid, 6.75 mcg of Vitamin B12,
d) 150 mcg of Selenium, 10 mg of Zinc, 120 mcg of Chromium, 2 mg of Copper, 4 mg of Manganese, 100 mcg of Iodine, 100 mcg of Molybdenum,
e) 200 mcg of Biotin, 500 - 1000 mg of Betaine, 100 mg of Oligoproanthocyanidins (OPC), 150 mg of Polyphenol complex
and possibly additional substances for the purpose of stabilisation and formulation of the above mentioned compounds of this claim.

The invention refers further to preparation of a mixture according to the immediately aforegoing section by first producing the said single compounds by chemical synthesis or isolating them from natural sources, therafter putting the single compunds together in a suitable vessel in relative amounts as to end up with the specified amount for each compund, then mixing the compounds after having put them together as aforementioned, and adding additional substances for stabilisation and/or formulation of this mixture.
The invention refers also to use of the same mixture for preparation of a food additive with beneficial effects on preventing, curing, or improving of symptoms of age related eye disease as well of use of this mixture for preparation of a medicament for preventing, improving, or curing of age related eye disease.

## Claims

1. Mixture of compounds comprising at least
a) 800 mcg (2664IU) of Vitamin A, 1000 mg of Vitamin C, 400IU of Vitamin E, 200 mcg of Vitamin K,
b) 10 mg of Beta carotene, 6 mg of Lutein, 5 mg of Lycopene, 100 mcg of Zeaxanthin,
c) 7.5 mg of Vitamin B1, 7.5 mg of Vitamin B2, 15 mg of Niacin, 15 mg of Pantothenic acid, 7.5 mg of Vitamin B6, 200 mcg of Folic acid, 6.75 mcg of Vitamin B12,
d) 200 mcg of Selenium, 10 mg of Zinc, 120 mcg of Chromium, 2 mg of Copper, 4 mg of Manganese, 100 mcg of Iodine, 100 mcg of Molybdenum,
e) 200 mcg of Biotin, 450 mg of Betaine, 100 mg of Oligoproanthocyanidins (OPC), 150 mg of Polyphenol complex, 600 to 5000 mg of mixed Omega 3 and 6, 4 to 6 g of Oligosaccharides (FOS) in particular oligo-fructose and/or beta glucan, 4 to 6 g of inulin,
and possibly additional substances for the purpose of stabilisation and formulation of the above mentioned compounds of this claim.

2. Preparation of a mixture according to claim 1 by first producing the said single compounds by chemical synthesis or isolating them from natural sources, therafter putting the single compunds together in a suitable vessel in relative amounts as to end up with the specified amount for each compund, then mixing the compounds after having put them together as aforementioned, and adding additional substances for stabilisation and/or formulation of this mixture.

3. Use of mixture of claim 1 and/or 2 for preparation of a food additive with beneficial effects preventing, curing, or improving of symptoms of diabetes.

4. Use of mixture of claim 1 and/or 2 for preparation of a medicament for preventing, improving, or curing of diabetes.

5. Mixture of compounds comprising at least
a) 800 mcg (2664IU) of Vitamin A, 500 mg of Vitamin C, 200IU of Vitamin E, 200 mcg of Vitamin K,
b) 10 mg of Beta carotene, 6 mg of Lutein, 5 mg of Lycopene, 100 mcg of Zeaxanthin,
c) 7.5 mg of Vitamin B1, 7.5 mg of Vitamin B2, 15 mg of Niacin, 15 mg of Pantothenic acid, 7.5 mg of Vitamin B6, 200 mcg of Folic acid, 6.75 mcg of Vitamin B12,
d) 200 mcg of Selenium, 10 mg of Zinc, 120 mcg of Chromium, 2 mg of Copper, 4 mg of Manganese, 100 mcg of Iodine, 100 mcg of Molybdenum,
e) 200 mcg of Biotin, 250 mg of Oligoproanthocyanidins (OPC), 150 mg of Polyphenol complex, 600 mg of Omega 3, 100 mg of Co-Q10
f) 1000 mg of Glucosamine
and possibly additional substances for the purpose of stabilisation and formulation of the above mentioned compounds of this claim.

6. Preparation of a mixture according to claim 5 by first producing the said single compounds by chemical synthesis or isolating them from natural sources, therafter putting the single compunds together in a suitable vessel in relative amounts as to end up with the specified amount for each compund, then mixing the compounds after having put them together as aforementioned, and adding additional substances for stabilisation and/or formulation of this mixture.

7. Use of mixture of claim 5 and/or 6 for preparation of a food additive with beneficial effects on preventing, curing, or improving of symptoms of osteo-arthritis.

8. Use of mixture of claim 5 and/or 6 for preparation of a medicament for preventing, improving, or curing of osteo-arthritis.

9. Mixture of compounds comprising at least
a) 800 mcg (2664IU) of Vitamin A, 500 mg of Vitamin C, 20 mcg of Vitamin D, 200 mcg of Vitamin K,
b) 10 mg of Beta carotene, 6 mg of Lutein, 5 mg of Lycopene, 100 mcg of Zeaxanthin,
c) 7.5 mg of Vitamin B1, 7.5 mg of Vitamin B2, 15 mg of Niacin, 15 mg of Pantothenic acid, 7.5 mg of Vitamin B6, 200 mcg of Folic acid, 6.75 mcg of Vitamin B12,
d) 200 mcg of Selenium, 10 mg of Zinc, 100 mg of Calcium, 50 mg of Magnesium,120 mcg of Chromium, 2 mg of Copper, 4 mg of Manganese, 100 mcg of Iodine, 100 mcg of Molybdenum,
e) 200 mcg of Biotin, 250 mg of Oligoproanthocyanidins (OPC), 150 mg of Polyphenol complex, 40 mg of Isofloavones in particular genistein and/or daidzein, 100 mg of Co-Q10,
f) 1000 mg of Glucosamine
and possibly additional substances for the purpose of stabilisation and formulation of the above mentioned compounds of this claim.

10. Preparation of a mixture according to claim 9 by first producing the said single compounds by chemical synthesis or isolating them from natural sources, therafter putting the single compunds together in a suitable vessel in relative amounts as to end up with the specified amount for each compund, then mixing the compounds after having put them together as aforementioned, and adding additional substances for stabilisation and/or formulation of this mixture.

11. Use of mixture of claim 9 and/or 10 for preparation of a food additive with beneficial effects for preventing, curing, or improving of symptoms of osteoporosis.

12. Use of mixture of claim 9 and/or 10 for preparation of a medicament for preventing, improving, or curing of osteoporosis.

13. Mixture of compounds comprising at least
a) 800 mcg (2664IU) of Vitamin A, 1000 mg of Vitamin C, 15 mcg of Vitamin D, 400IU of Vitamin E, 200 mcg of Vitamin K,
b) 10 mg of Beta carotene, 6 mg of Lutein, 5 mg of Lycopene, 100 mcg of Zeaxanthin,
c) 7.5 mg of Vitamin B1, 7.5 mg of Vitamin B2, 15 mg of Niacin, 15 mg of Pantothenic acid, 7.5 mg of Vitamin B6, 200 mcg of Folic acid, 6.75 mcg of Vitamin B12,
d) 200 mcg of Selenium, 10 mg of Zinc, 100 mg of Calcium, 50 mg of Magnesium,120 mcg of Chromium, 2 mg of Copper, 4 mg of Manganese, 100 mcg of Iodine, 100 mcg of Molybdenum,
e) 200 mcg of Biotin, 1000 mg of Betaine, 250 mg of Oligoproanthocyanidins (OPC), 150 mg of Polyphenol complex, 500 mg of curcuminoids, 100 mg of ginkgo biloba, 600 to 10 000 mg of mixed Omega 3, 60 - 90 mg of Co-Q10,
and possibly additional substances for the purpose of stabilisation and formulation of the above mentioned compounds of this claim.

14. Preparation of a mixture according to claim 13 by first producing the said single compounds by chemical synthesis or isolating them from natural sources, therafter putting the single compunds together in a suitable vessel in relative amounts as to end up with the specified amount for each compund, then mixing the compounds after having put them together as aforementioned, and adding additional substances for stabilisation and/or formulation of this mixture.

15. Use of mixture of claim 13 and/or 14 for preparation of a food additive with beneficial effects on preventing, curing, or improving of symptoms of asthma.

16. Use of mixture of claim 13 and/or 14 for preparation of a medicament for preventing, improving, or curing of asthma.

17. Mixture of compounds comprising at least
a) 800 mcg (2664IU) of Vitamin A, 500 mg of Vitamin C, 200IU of Vitamin E, 200 mcg of Vitamin K,
b) 10 mg of Beta carotene, 6 mg of Lutein, 5 mg of Lycopene, 100 mcg of Zeaxanthin,
c) 7.5 mg of Vitamin B1, 7.5 mg of Vitamin B2, 15 mg of Niacin, 15 mg of Pantothenic acid, 7.5 mg of Vitamin B6, 200 mcg of Folic acid, 6.75 mcg of Vitamin B12,
d) 200 mcg of Selenium, 10 mg of Zinc, 120 mcg of Chromium, 2 mg of Copper, 4 mg of Manganese, 100 mcg of Iodine, 100 mcg of Molybdenum,
e) 200 mcg of Biotin, 1000 mg of Betaine, 250 mg of Oligoproanthocyanidins (OPC), 150 mg of Polyphenol complex, 40 mg of Isofloavones in particular genistein and/or daidzein , 600 mg of Omega 3, 4 to 6 g of Oligosaccharides (FOS) in particular oligo-fructose and/or beta glucan, 4 to 6 g of inulin, 60 - 90 mg of Co-Q10,
and possibly additional substances for the purpose of stabilisation and formulation of the above mentioned compounds of this claim.

18. Preparation of a mixture according to claim 17 by first producing the said single compounds by chemical synthesis or isolating them from natural sources, therafter putting the single compunds together in a suitable vessel in relative amounts as to end up with the specified amount for each compund, then mixing the compounds after having put them together as aforementioned, and adding additional substances for stabilisation and/or formulation of this mixture.

19. Use of mixture of claim 17 and/or 18 for preparation of a food additive with beneficial effects on preventing, curing, or improving of symptoms of mental decline.

20. Use of mixture of claim 17 and/or 18 for preparation of a medicament for preventing, improving, or curing of mental decline.

21. Mixture of compounds comprising at least
a) 800 mcg (2664IU) of Vitamin A, 500 mg of Vitamin C, 265 mg (400IU) of Vitamin E, 50 mcg of Vitamin K,
b) 10 mg of Beta carotene, 6 mg of Lutein, 5 mg of Lycopene, 100 mcg of Zeaxanthin,
c) 7.5 mg of Vitamin B1, 7.5 mg of Vitamin B2, 15 mg of Niacin, 15 mg of Pantothenic acid, 7.5 mg of Vitamin B6, 200 mcg of Folic acid, 6.75 mcg of Vitamin B12,
d) 150 mcg of Selenium, 10 mg of Zinc, 120 mcg of Chromium, 2 mg of Copper, 4 mg of Manganese, 100 mcg of Iodine, 100 mcg of Molybdenum,
e) 200 mcg of Biotin, 500 - 1000 mg of Betaine, 100 mg of Oligoproanthocyanidins (OPC), 150 mg of Polyphenol complex
and possibly additional substances for the purpose of stabilisation and formulation of the above mentioned compounds of this claim.

22. Preparation of a mixture according to claim 21 by first producing the said single compounds by chemical synthesis or isolating them from natural sources, therafter putting the single compunds together in a suitable vessel in relative amounts as to end up with the specified amount for each compund, then mixing the compounds after having put them together as aforementioned, and adding additional substances for stabilisation and/or formulation of this mixture.

23. Use of mixture of claim 21 and/or 22 for preparation of a food additive with beneficial effects on preventing, curing, or improving of symptoms of age related eye disease.

24. Use of mixture of claim 21 and/or 22 for preparation of a medicament for preventing, improving, or curing of age related eye disease.
